# EUROPÄISCHE PATENTANMELDUNG

(11) **EP 0 881 494 A1**
(43) Veröffentlichungstag der Anmeldung: **02.12.1998**
(21) Anmeldenummer: 98107690.4
(22) Anmeldetag: 28.04.1998
(51) Int. Cl.: G01N 33/72, G01N 33/68

(54) **Verfahren zur simultanen Bestimmung von Proteinen bzw. entsprechenden Derivaten**

(30) Priorität: 29.04.1997 DE 19718019; 08.08.1997 DE 19734325
(71) Anmelder: BOEHRINGER MANNHEIM GMBH, 68298 Mannheim (DE)
(72) Erfinder: Kobold, Uwe, Dr., 82407 Wielenbach (DE); Wolf, Peter, Dr., 82380 Peissenberg (DE); Engel, Wolf-Dieter, Dr., 82340 Feldafing (DE)

(57) **Zusammenfassung**

Die Erfindung betrifft Verfahren zur quantitativen simultanen Bestimmung von Proteinen, wie beispielsweise glykierten und/oder nicht glykierten Proteinen anhand massenspektrometrischer Methoden. Die gewonnenen Meßdaten bzw. entsprechende Verhältniswerte können für präzisere diagnostische Aussagen, insbesondere in Zusammenhang mit Diabetes, verwendet werden.

## Beschreibung

Die Erfindung betrifft ein Verfahren zur simultanen quantitativen Bestimmung von Proteinen, glykierten Modifikationen bzw. hiervon abgeleiteten Derivaten, wie beispielsweise deren Alterungsprodukte (sogen. AGE, advanced glycosilation end products) in einer wäßrigen Probe. Das Verfahren eignet sich insbesondere für Hämoglobin (Hb) und dessen Derivate bzw. entsprechende möglichst homogene Glykoproteinfraktionen (z.B. gHb, HbA1c und Hb-AGE). Die Quantifizierung erfolgt durch Bestimmung der relativen Konzentrationsverhältnisse aus den jeweils gemessenen analytspezifischen Signalen.

Zur Bestimmung von Proteinen, wie glykierten Proteinen gibt es eine Vielzahl von Verfahren. Diese lassen sich prinzipiell in drei Gruppen einteilen, und zwar in Abhängigkeit von der Art und Weise wie glykierte und nicht glykierte Proteinkomponenten getrennt und quantifiziert werden (Clin. Chemistry 32 (1986), B64 - B70). Als erste Gruppe sind physikalisch-chemische Methoden, basierend auf der Nutzung von Ladungsunterschieden, zu nennen. Als zweite Gruppe sind Methoden anzuführen, welche die unterschiedliche chemische Reaktivität von glykiertem und nicht glykiertem Protein ausnutzen. Drittens sind immunologische Verfahren zu nennen. In jüngster Zeit hat sich insbesondere ein Verfahren als vorteilhaft erwiesen bei dem die jeweilige Proteinprobe zunächst einer proteolytischen Spaltung unterzogen wird und die Spaltprodukte anschließend über HPLC getrennt werden (EP 0693559, U. Kobold et al., Proc. XVI ICCC London 1996, C25, S. 374). Keine der beschriebenen Methoden erlaubt es jedoch, mehrere der beschriebenen Analyten gleichzeitig aus einer Lösung zu bestimmen. Die meisten der bekannten Verfahren, mit Ausnahme der immunologischen Verfahren, zeigen zudem nur eine geringe Spezifität. Beispielsweise sind für AGE-Proteine derzeit noch keine ausreichend spezifischen Testverfahren verfügbar, insbesondere deshalb nicht, da die Spezifität der Antikörper in der Regel nicht bekannt ist bzw. entsprechende Referenzmethoden fehlen.

Für glykierte Proteine mit geringem Molekulargewicht, wie gHb, wurde unter bestimmten Bedingungen darüber hinaus die Detektion anhand massenspektrometrischer Maßnahmen beschrieben (N.B. Roberts et al., Abstract / Poster, Proc. XVI ICCC London 1996, C28, S. 375). Dieses Verfahren ist jedoch ausschließlich auf native Proben ausgerichtet, was die Bestimmung von komplexeren Proteinmischungen bedeutend einschränkt, wenn nicht unmöglich macht.

Mit keinem der derzeit bekannten Verfahren ist es jedoch möglich, verschiedene Analyten entsprechender Proteine, insbesondere auch solche unterschiedlicher Molekulargewichte, die zudem in derselben Probenlösung vorliegen können, simultan, direkt und mit ausreichend hoher Spezifität zu bestimmen. Der Erfindung lag somit die Aufgabe zugrunde, ein entsprechendes Bestimmungsverfahren zur Verfügung zu stellen.

Die Aufgabe wird gelöst durch ein Verfahren zur Bestimmung von Proteinen, glykierten Proteinen und/oder entsprechenden Derivaten, wie beispielsweise von Alterungsprodukten glykierter Proteine (AGEs), in einer wäßrigen Probe, dadurch gekennzeichnet, daß die Messung massenspektrometrisch erfolgt und bei der Messung mindestens zwei Komponenten einer entsprechenden Proteinmischung gleichzeitig bestimmt werden. Die Proteinmischung kann glykierte sowie nicht glykierte Proteine unterschiedlichen Molekulargewichts, wie beispielsweise Antikörperfragmente, und insbesondere auch solche mit höherem Molekulargewicht (> 30.000 Da) enthalten. Besonders vorteilhaft hat sich das erfindungsgemäße Verfahren erwiesen, wenn drei oder mehr Komponenten nebeneinander bestimmt werden sollen. Ferner hat sich als vorteilhaft erwiesen, wenn zwei oder mehr glykierte Proteinkomponenten nebeneinander bestimmt werden sollen. Das Verfahren ist dabei zahlenmäßig nicht auf eine Obergrenze von Komponenten beschränkt. So können auf diese Weise beispielsweise ca. 30 oder auch bis 50 Komponenten nebeneinander bestimmt werden, abhängig vom jeweiligen Molekulargewicht und der Massendifferenzen der einzelnen Komponenten. Die massenspektrometrische Messung kann erfindungsgemäß anhand eines entsprechenden Spray-Verfahrens, wie Elektrospray-Ionisation oder Atmosphärendruck-Ionisation, über Laserdesorption oder ein gleichwertiges Ionisationsverfahren erfolgen. In der Regel läuft das massenspektrometrische Verfahren automatisiert ab und kann direkt datentechnisch (on-line) ausgewertet werden. Die gewonnenen Meßdaten können nach entsprechender Verhältnisbildung direkt für entsprechende Aussagen in der klinischen Diagnostik verwendet werden.

Maßnahmen für die Probenvorbereitung können zum einen lediglich in der Verdünnung der Probe bzw. Probenlösung mit einem für die Massenspektrometrie geeigneten Lösungsmittel bestehen. Für die Massenspektroskopie geeignete Lösungsmittel sind beispielsweise organische Säuren (Essigsäure, Ameisensäure), Wasser oder organische Lösungsmittel (Methanol, Acetonitril oder ähnliche). Zum anderen hat sich als vorteilhaft erwiesen, wenn die Probenlösung zuvor einer chemischen oder enzymatischen Spaltung unterzogen wird, d.h. z.B. mit einem proteolytischen Enzym in einem geeigneten Puffersystem vorbehandelt wird. Entsprechend geeignete Enzyme sind beispielsweise Trypsin, Chymotrypsin, Endoproteinase Lys C und/oder Endoproteinase Glu-C. Ferner hat sich als vorteilhaft erwiesen, wenn die Probenlösung vor der masserspektrometrischen Messung von nicht proteinogen Bestandteilen abgetrennt bzw. beispielsweise chromatographisch oder immunologisch gereinigt wird. Dabei kann die Aufreinigung on-line oder off-line zur Massenspektroskopie erfolgen.

Als Probenmaterial kommen in erster Linie Flüssigkeiten menschlichen oder tierischen Ursprungs, wie Vollblut, Blut, Plasma oder Serum, aber auch Gewebe, Ascites oder pflanzliche Extrakte in Betracht. Darüber hinaus ist es möglich das erfindungsgemäße Verfahren für solche Lösungen einzusetzen, denen eine oder mehrere Proteinkomponenten künstlich zugesetzt wurden (z.B. Standard-Lösungen). Bei den zu bestimmenden Probenbestandteilen handelt es sich um nicht glykierte Proteine, wie beispielsweise Albumin monoklonale oder polyklonale Antikörper, Fab- oder Fc-Fragmente, Hämoglobin und dessen Derivate, und/ oder glykierte Proteine und deren Derivate, wie beispielsweise glykiertes Albumin, gHB, HbA1c oder Hb-AGE. Die Proteine der Probenmischung können darüber hinaus geringfügig und/oder beträchtlich unterschiedliche Molekulargewichte aufweisen. So können beispielsweise Proteine mit unterschiedlichem Glykierungsgrad bereits innerhalb enger Molekulargewichtsbereiche bestimmt werden. Ferner können entsprechende Mischungen darüber hinaus eine Reihe anderer Proteine, auch weit höheren Molekulargewichts, enthalten.

Im folgenden wird das erfindungsgemäße Verfahren, das insbesondere darin besteht, daß verschiedene Proteinkomponenten mit Hilfe der Massenspektrometrie (MS) simultan und quantitativ bestimmt werden, anhand möglicher Varianten (Variante A und B) detaillierter beschrieben.

Bei der ersten Variante, Variante A, erfolgt die Messung beispielsweise direkt aus verdünntem Vollblut. Mit Elektrospray- MS werden Meßsignale von den nicht glykierten Proteinen und den glykierten Proteinen bzw. den Alterungsprodukten erhalten. Zum Beispiel können so nebeneinander Hämoglobin (Hb), glykiertes Hämoglobin (gHb und HbA1c) und advanced glycation end product von Hb (Hb-AGE) oder Serum-Albumin (HSA), glykiertes HSA (gHSA) und HSA-AGE bestimmt werden. Insbesondere hat sich das erfindungsgemäße Verfahren als geeignet erwiesen, wenn die Glykierung der genannten Proteine auf nicht enzymatische Weise erfolgt ist. Die Rohdaten können bevorzugt direkt in einer Messung erhalten werden. Aus den Intensitätsverhältnissen der analytspezifischen Signale, welche mit den Konzentrationsverhältnissen korrelieren, werden die relativen Anteile der Einzelkomponeten in der Probe ermittelt. Zur Ermittlung der jeweiligen Responsefaktoren können Kalibratoren verwendet werden.

Im einzelnen wird dabei wie folgt verfahren: Das Probenmaterial, insbesondere hämolysiertes Vollblut wird mit einem geeigneten Lösungsmittel, zum Beispiel Gemische aus flüchtigen organischen Säuren (Essigsäure, Ameisensäure), Wasser und einem organischen Lösungsmittel (Methanol, Acetonitril), verdünnt und die erhaltene Lösung direkt für die massenspektrometrische Messung verwendet. Alternativ zur direkten Messung kann die Probe auch über einen chromatographischen oder immunsorpiven Aufreinigungsschritt vorbehandelt werden, wobei die Vorbehandlung on-line zur massenspektrometrischen Messung erfolgen kann.

Bei einer möglichen zweiten Variante, Variante B, erfolgt vor der massenspektrometrischen Messung eine enzymatische oder chemische Spaltung der Probe. Das Probenmaterial kann entsprechend zu Variante A beispielsweise Vollblut sein. Bei der enzymatischen oder chemischen Spaltung werden analytspezifische Peptidfragmente erzeugt. Die Intensitätsverhältnisse der spezifischen Peptide werden bestimmt und daraus die relativen Anteile der Analyten berechnet. Zur Ermittlung der jeweiligen Responsefaktoren können Kalibratoren verwendet werden.

Im einzelnen wird dabei wie folgt verfahren: Das Probenmaterial, beispielsweise hämolysiertes Vollblut, wird mit einem proteolytischem Enzym in einem geeigneten Reaktionspuffer inkubiert. Die Hämolyse erfolgt nach bekannten Methoden. Als Enzym haben sich hier insbesondere solche Proteasen als geeignet erwiesen, die befähigt sind, die zu bestimmenden Proteine spezifisch zu spalten. Neben Trypsin haben sich Chymotrypsin, Endoproteinase Lys C und/oder Endoproteinase Glu-C als besonders geeignet erwiesen. Die proteolytischen Enzyme werden üblicherweise - abhängig von der jeweiligen Probe - in einer Konzentration von 1/200 bis 1/10, vorteilhafterweise in Konzentrationen von ungefähr 1/100 der Gewichtsmenge Protein eingesetzt.

Geeignete Reaktionspuffer sind beispielsweise Ammoniumcarbonat-Puffer und/oder Natriumphosphat-Puffer, die üblicherweise in Konzentrationen von ungefähr 0,5 mM bis 200 mM im pH-Bereich von 3.0 bis 9.0 eingesetzt werden und gegebenenfalls - abhängig von der jeweiligen Protease - durch weitere Hilfsstoffe, wie Aktivatoren, und/oder Stabilisatoren, z.B. Natriumdodecylsulfat, Harnstoff, Guanidinhydrochlorid, Komplexbildner (z.B. Kohlenmonoxid, Cyanidionen und/oder EDTA), Antioxidantien, Konservierungsmittel oder Acetonitril ergänzt werden können. Als vorteilhaft hat sich erwiesen, wenn der Puffer eine Konzentration zwischen 10 mM und 50 mM und einen pH-Wert von 4,2 bis 7,8 aufweist. In diesem pH-Bereich erweisen sich in der Regel insbesondere die nicht enzymatisch glykierten Proteine als stabil.

Die Inkubation mit dem jeweiligen proteolytischen Enzym erfolgt zwischen ca. 20°C und 40°C, vorteilhafterweise bei ca. 25°C und kann je nach Enzym nach einigen Minuten oder auch Stunden oder sogar mehreren Tagen beendet werden. Als günstig haben sich Verdauzeiten von bis zu 24 Stunden erwiesen.

Die durch Enzymbehandlung erhaltene Lösung enthält neben anderen Fragmenten, die für die jeweils zu bestimmenden Proteine spezifischen Peptide und deren glykierte Varianten, beziehungsweise deren AGE-Derivate. Die Peptidgemische werden anschließend in einem HPLC-Verfahren (z.B. Reverse-phase-Materialien) aufgetrennt. Solche Trennverfahren sind dem Fachmann bekannt (z.B. K.L. Stone, J.I. Elliott, G. Peterson, W. McMurray, K.R. Williams, "Reversed-phase high performance liquid chromatography for fractionation of enzymatic digests and chemical cleavage products of proteins", Methods in Enzymology, 193 (1990) 389-412). Alternativ kann auch mit anderen Trennverfahren, wie z.B. Kapillarelektrophorese gearbeitet werden (z.B. Capillary Electrophoresis Separations of Peptides: Practical Aspects and Applications, Chap. 9, S.237-271 in Capillary Electrophoresis, Academic Press 1992). Das Eluat aus dem Trennverfahren (HPLC oder CE) wird direkt in die Ionenquelle des Massenspektrometers geleitet. Bei Verwendung von Tandem-MS-Techniken (MS/MS, MSⁿ) kann auch auf die chromatographische Vortrennung verzichtet werden (Analyt. Chemistry 68 (1996), 527-533).

Aus den gemessenen Signalintensitäten des glykierten, AGE und des jeweiligen nichtglykierten Peptidanteils ist die gleichzeitige selektive Bestimmung von glykiertem, AGE und nichtglykiertem Protein möglich. Bei der rechnerischen Auswertung wird der prozentuale Anteil von glykiertem und AGE-Protein bezogen auf nicht glykiertes Protein erhalten. Insbesondere ist das Verfahren somit für die Bestimmung von glykiertem Hämoglobin und Hämoglobin-AGE neben nicht glykiertem Hämoglobin von Vorteil, da die Konzentrationen sämtlicher Komponenten gleichzeitig und zudem quantitativ ermittelt werden können.

Zur Aufnahme eines Massenspektrums von Proteinen und Proteinderivaten für das erfindungsgemäße Verfahren sind prinzipiell dem Stand der Technik entsprechende Maßnahmen geeignet, und zwar insbesondere "weiche" Ionisationsverfahren, wobei die Verfahren der "atmospheric pressure ionization" (API) bzw. "electrospray ionization" (ESI) besonders geeignet sind. Zur Massenbestimmung der bei der Ionisation gebildeten Ionen können grundsätzlich alle mit diesem Ionisationsverfahren koppelbaren Massenanalyser verwendet werden, zum Beispiel Quadrupolmassenspektrometer, Flugzeitmassenspektrometer, Ionenfallen oder Sektorfeldgeräte. Die Massenspektrometer können zur Verbesserung der Selektivität oder Empfindlichkeit auch im MS/MS oder MSⁿ Modus betrieben werden.
Als Ionisationsverfahren hat sich darüber hinaus die Matrix unterstützte Laserdesorptions-Ionisation (MALDI) als geeignet erwiesen.

Die Messung erfolgt bevorzugt mit positiv geladenen Ionen. Zur Messung der Probe mit positiv geladenen Ionen mit ESI wird das Reaktionsgemisch zum Beispiel in sauren Lösungsmitteln gelöst, wie beispielsweise Essigsäure oder Ameisensäure, der Zusatz von organischen Lösungsmitteln, wie Acetonitril oder Methanol von 40 bis zu 90%, ist vorteilhaft, jedoch nicht zwingend erforderlich. Diese Lösung wird dem Ionisationssystem mit Flußraten zwischen 10 nl/min und 1 ml/min zugeführt. Alternativ kann auch die sogenannte Nanospray-Technologie verwendet werden, welche auch mit rein wäßrigen Lösungen gute Signalausbeuten liefert. Die Bildung der Ionen erfolgt durch Zerstäuben der Lösung in einem elektrischen Feld, wobei der Prozeß pneumatisch durch Zufuhr eines Gases oder thermisch unterstützt werden kann. Die gebildeten Ionen werden im elektrischen Feld fokusiert und über geeignete Blenden oder Kapillaren in den Hochvakuum-Bereich des Massenspektrometers überführt. Unter diesen Ionisationsbedingungen entstehen typischerweise Ionenserien von einfach und mehrfach protonierten Molekülen, deren jeweiliges Verhältnis Masse zu Ladung (m/z) mit beispielsweise einem Quadrupol-Massenanalyser bestimmt wird. Aus den erhaltenen Ionenserien läßt sich das Molekulargewicht der in der Probe enthaltenen Komponenten berechnen. Die Berechnung erfolgt üblicherweise mit kommerziell verfügbaren Software-Paketen, wie beispielsweise ICIS Biomass von Finnegan MAT. Die Intensitäten der erhaltenen Ionen korrelierten mit den Konzentration der jeweiligen Komponenten in der Lösung.

Geeignete Geräte zur Aufnahme entsprechender Massenspektren sind beispielsweise von den Firmen Finnigan MAT, Perkin Elmer Sciex, Bruker, MicroMass und anderen kommerziell erhältlich.

Alternativ zur direkten Massenbestimmung der in der Probe enthaltenen Proteinkomponenten, d.h. anstatt des Einsatz eines kompletten Gemischs, kann vor der Messung eine vollständige oder teilweise Probenaufreinigung oder Anreicherung durchgeführt werden. Hier kommen bevorzugt Filtrationsprozesse (Ultrafiltration) oder chromatographische Reinigungsverfahren zum Einsatz. Insbesondere die chromatographischen Verfahren sind automatisierbar und können somit als direkte Kopplungsverfahren mit der Massenspektrometrie verwendet werden. Geeignete chromatographische Verfahren sind zum Beispiel Reversed Phase-, Ionenaustauscher-, Gelpermeations- oder Affinitätschromatographie. Darüber hinaus haben sich Elektrophorese, insbesondere kapillarelektrophorese Verfahren als geeignet erwiesen.

Mit der Bestimmung von beispielsweise glykierten Proteinen, wie von HbA1c, im Verhältnis zu nicht glykierten Formen werden heute insbesondere wichtige Beiträge zur Ermittlung des jeweiligen Diabetestyps bzw. -grades geleistet. Das erfindungsgemäße Verfahren erlaubt es nun erstmals unterschiedliche Proteine verschiedenen Glykierungsgrades gleichzeitig zu messen und zu interpretieren bzw. die erhaltenen Werte rechnerisch zueinander ins Verhältnis zu setzen. Dieses Vorgehen ist insbesondere in der Klinischen Diagnostik von Bedeutung, indem beispielsweise Trends in der Entwicklung des glykämischen Zustandes der Vergangenheit, wie z.B. von Diabetespatienten, aufgezeigt sowie überhaupt auf existierende krankhafte Zustände, wie beispielsweise Nephropathie, oder auf Unterschiede in der Glykierung, die vom Diabetestyp abhängen, hingewiesen werden können. Darüber hinaus bietet das erfindungsgemäße Verfahren den Vorteil, daß es praktisch leicht zu realisieren und ökonomisch ist, da die verschiedenen, gegebenenfalls glykierten Proteine einer Probe nebeneinander mit demselben analytischen Instrumentarium bestimmt werden können. Durch Online-Datenerfassung wird das Verfahren weiter erleichtert.

Erläuterung zu den Abbildungen:

### Abbildung 1

Das Massenspektrogramm (Intensität (I) / Molekulargewicht (MG)) von nativem Haemoglobin (Probe 1), welches eine so gut wie reine, nicht modifizierte alpha-Kette aufweist (gemessen MG 15.124 Da, erwartet MG 15.126 Da).

### Abbildung 2

Das Massenspektrum (I/MG)von Haemoglobin, welches ½ Woche mit Glucose inkubiert wurde (Probe 2), zeigt eine mehrfache Glykierung, bis zu vierfach, und darüber hinaus Carboxymethylierungsprodukte (AGE).

### Abbildung 3

Das Massenspektrum (I/MG) von Haemoglobin, welches eine Woche mit Glucose inkubiert wurde (Probe 3), zeigt sowohl eine verstärkte Glykierung, als auch verstärkte Carboxymethylierung.

### Abbildung 4

Massenspektrometrisch bestimmtes Verhältnis von glykiertem Albumin ((glc)₂ HSA) zu Albumin (HSA) in verschiedenen Patientenproben im Vergleich zum auf HSA bezogenen Fructosamingehalt.

### Abbildung 5

Massenspektrum (I/MG) von 〈CEA〉-monoklonalem Antikörper (MAK), Fab-Fragmente, nicht modifiziert (MG 48003 Da).

### Abbildung 6

Massenspektrum (I/MG) von MAK 〈CEA〉, Fab-(biotin)ₙ mit n gleich 1 und n gleich 2 (MG 48003 plus n x 512 Da).

### Abbildung 7

Massenspektrum (I/MG) von MAK 〈CEA〉, Fab-(biotin)ₙ mit n gleich Null, 1, 2, 3 und 4 (MG 48003 plus n x 512 Da).

### Abbildung 8

Massenspektun (I/MG) von MAK 〈TSH〉 F(ab')₂-(biotin)ₙ mit n gleich 1 und n gleich 2 (MG 99107 plus n x 512 Da).

Die verwendeten Abkürzungen haben folgende Bedeutungen:
- α:: alpha-Kette von Hämoglobin
- glc:: ein Glucose-Molekül als Ketoamin gebunden
- (glc)ₙ:: n-fach als Ketoamin gebundene Glucose-Moleküle, wobei n eine ganze Zahl von 0 bis 5 bedeutet
- CM:: eine Carboxymethyl-Gruppe gebunden an Lysin oder N-terminales Valin.
- Fab, F(ab')₂: Antikörperfragmene

Die folgenden Beispiele erläutern die Erfindung weiter:

### Beispiel 1

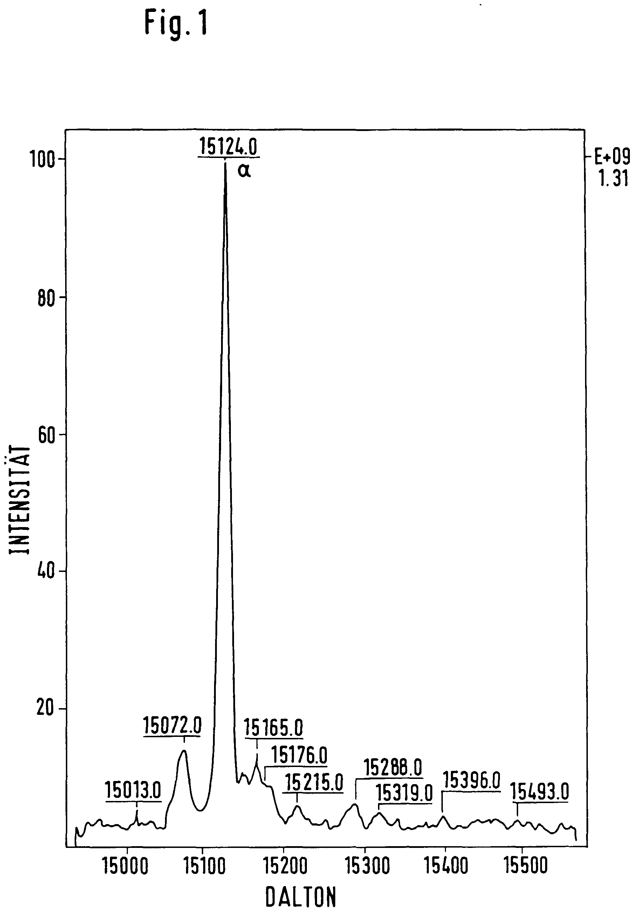

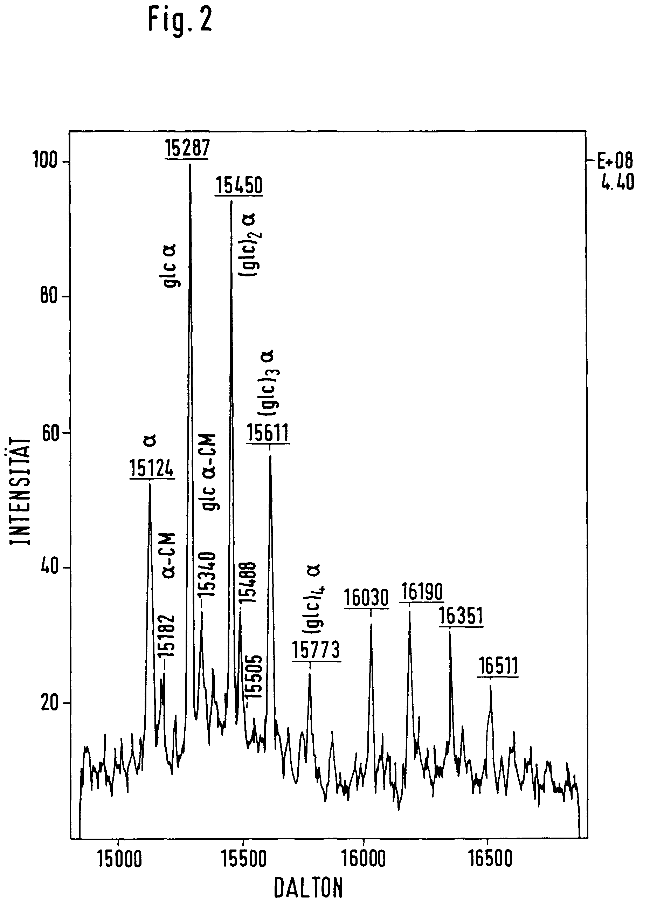

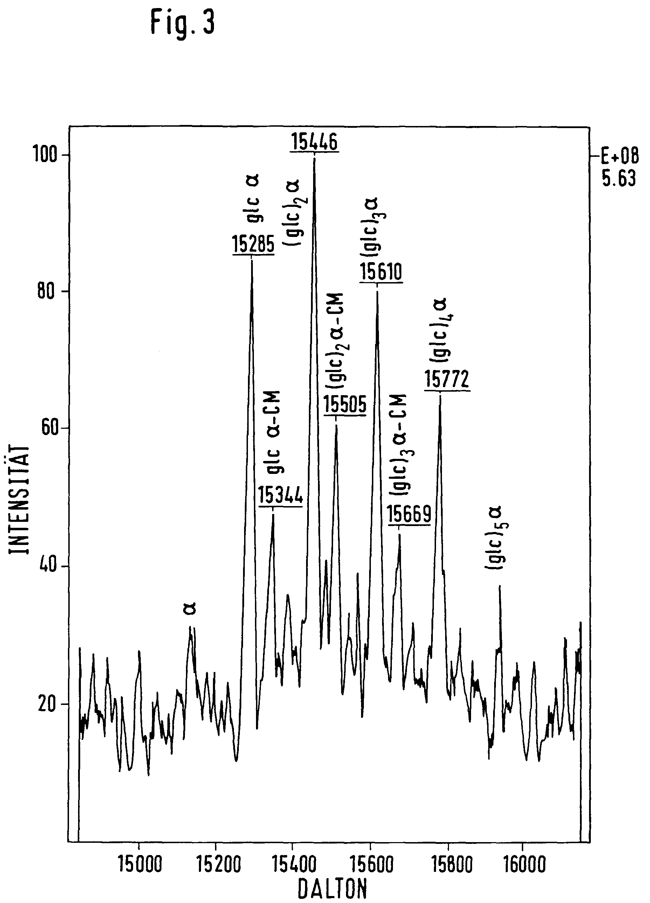

Verschiedene Hämoglobin-Proben aus humanem Erythrozyten-Hämolysat wurden in 0.5 %iger wäßriger Ameisensäure gelöst. Dabei wurde eine Konzentration von ca. 0.2 mg/ml Gesamt-Hb eingestellt. Von dieser Lösung wurden 5 µL auf eine Reversed Phase HPLC Säule, (PLRP-S 1000 Angström, 1 x 75 mm) aufgegeben und mit einem Gradienten von 0.5 % Ameisensäure in Wasser bis zu einer Lösung bestehend aus 0.5 % Ameisensäure/ 20 % Wasser/80 % Acetonitril bei einem Fluß von 50 µL/min eluiert. Die HPLC- Säule wurde on-line mit einer Elektrospray-Ionenquelle verbunden (Massenspektrometer Finnigan MAT SSQ700 mit Finnigan API Ionenquelle). Die zu bestimmenden Hämoglobine eluierten dabei innerhalb von 10 min von der Säule. Die Parameter des Massenanalysators wurden so gewählt, daß alle in der Ionenquelle erzeugten Ionen mit einem Verhältnis von Masse zur Ladung (m/z) zwischen 600 und 1.400 nachgewiesen wurden. Die erhaltenen Rohdaten wurden mit dem ICIS Biomass-Auswerteprogramm (Finnigan MAT) ausgewertet und die m/z-Rohdaten in eine Molekulargewichts-Scala transformiert (Abbildungen 1 bis 3).

### Probenmaterial:

Probe 1: Hämolysat von Erythrozyten aus nativem Vollblut
Probe 2: Hämolysat inkubiert mit Glucose ½ Woche bei 35°C
Probe 3: Hämolysat inkubiert mit Glucose 1 Woche bei 35°C

Zum Vergleich der unterschiedlichen Proben wurden jeweils die Signale der alpha-Kette des Hämoglobins ausgewertet.

Die erhaltenen Massenspektren zeigen deutlich, daß bei Behandlung mit Glucose der Anteil von glykiertem Hämoglobin (gHb) in der Probe ansteigt, wobei auch mehrfach Glykierungen auftreten. Zusätzlich zu den Glykierungsprodukten sind auch AGE-Signale, insbesondere durch Carboxymethylierung verursacht, zu beobachten.

### Ergebnis:

Probe 1: Natives Hämoglobin zeigt nahezu nicht modifizierte alpha-Kette
Probe 2: Inkubation über ½ Woche zeigt mehrfach Glykierung bis zu vier Addukten pro alpha-Kette, im Mittel 1.5 und zusätzliche Carboxymethylierung (AGE)
Probe 3: Inkubation über 1 Woche zeigt verstärkte Glykierung bis zu fünf Addukten pro alpha-Kette, im Mittel 2.5 und verstärkte Carboxymethylierung.

Hb-AGE läßt sich als carboxymethyliertes Hemoglobin in Hemoglobin-Präparationen direkt mit ESI-MS nachweisen.

### Beispiel 2

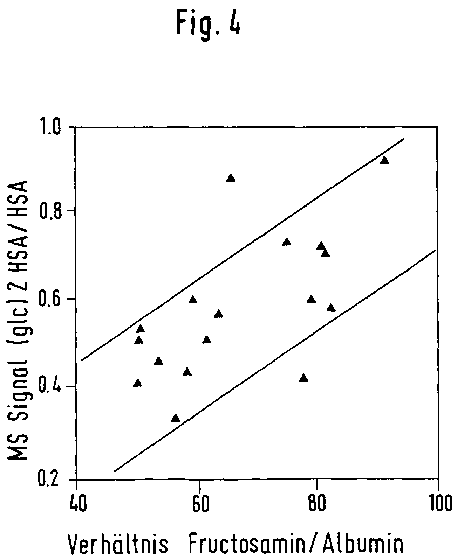

Verschiedene Humaserum Proben (gesunde und gut eingestellte Diabetiker, HbA1c zwischen 4 und 6%), welche mit dem in der klinischen Chemie gebräuchlichen Fructosamin-Test hinsichtlich des Gehaltes an glykiertem Gesamtprotein charakterisiert wurden, wurden zur massenspektrometrischen Bestimmung des Verhältnisses von glykiertem zu nicht glykiertem Serum-Albumin verwendet. Die Serum-Proben wurden im Verhältnis 1:100 mit Wasser verdünnt. Von dieser Lösung wurden 5 µL auf eine Reversed Phase HPLC-Säule, PLRP-S 1000 Angström, 1 x 75 mm, aufgegeben und die enthaltenen Proteine mit einer Lösung von 0.5 % Ameisensäure/ 20 % Wasser/ 80% Acetonitril bei einem Fluß von 50 µL/min eluiert. Die HPLC-Säule wurde on-line mit einer Elektrospray Ionenquelle verbunden (Massenspektrometer Finnigan MAT SSQ700 mit Finnigan API Ionenquelle). Die zu bestimmenden Albumine eluierten dabei innerhalb von 10 min von der Säule. Die Aufnahmeparameter des Massenspektrometers wurden so gewählt, daß die fünfzigfach protonierten Ionen der zu bestimenden Albuminderivate detektiert wurden. Dies entsprich einem Messbereich von m/z 1320 bis m/z 1350. Ausgewertet wurden die Intensitäten der Signale, welche dem unmodifizierten Albumin beziehungsweise dem zweifach glykiertem Albumin zuzuordnen sind. In Abbildung 4 ist für siebzehn Humanserum-Proben dargestellt, wie sich die massenspektrometrisch gemessenen Intensitätsverhältnisse von zweifach glykiertem Albumin zu Albumin (y-Achse) zum Quotienten aus Fructosamin zu Albumin Messwerten [µmol/l : g/dl] (x-Achse) verhalten. Wie erwartet wird ein Anstieg des Gehaltes an glykiertem Albumin mit steigendem Fructosamingehalt beobachtet. Bedingt durch die unterschiedliche Spezifität beider Verfahren (im Fructosamin-Test werden alle glykierten Serumproteine erfaßt, bei der massenspektroskopischen Bestimmung im vorliegenden Beispiel nur das Albumin) besteht zwischen den beiden Methoden lediglich eine grobe Korrelation.

### Beispiel 3

Verschiedene Antikörper-Derivate wie Fab- und F(ab')₂-Fragmente verschiedener Antikörper, welche in unterschiedlichem Ausmaß mit einem Biotinreagenz (Biotinoyl-amino-3,6-dioaoctanylaminocarbonylheptansäure-N-hydroxysuccinimidester, Biotin-DDS-Derivat) modifiziert wurden, wurden entsprechend dem in Beispiel 1 beschriebenen Verfahren untersucht. Die Proben wurden in 0.5%iger wäßriger Ameisensäure gelöst. Dabei wurde eine Konzentration von ca. 5 picomol/microliter eingestellt. Von dieser Lösung wurden 5 µL auf eine Reversed Phase HPLC Säule, PLRP-S 1000 Angström 1 x 75 mm, aufgegeben und mit einem Gradienten von 0.5% Ameisensäure in Wasser nach 0.5% Ameisensäure/ Acetonitril bei einem Fluß von 50 µL/min eluiert. Die HPLC-Säule wurde on-line mit einer Elektrospray Ionenquelle verbunden (Massenspektrometer Finnigan MAT SSQ700 mit Finnigan API Ionenquelle). Die zu bestimmenden Substanzen eluierten dabei innerhalb von 10 min von der Säule. Die Elektrospray-Massenspektren wurden im scan modus von m/z 600 bis 3000 aufgenommen. Aus den erhaltenen Rohdaten wurden mit dem ICIS-Biomass-Auswerteprogramm (Finnigan MAT) ausgewertet und die m/z Rohdaten in eine Molekulargewichts-Scala transformiert.

### Probenmaterial:

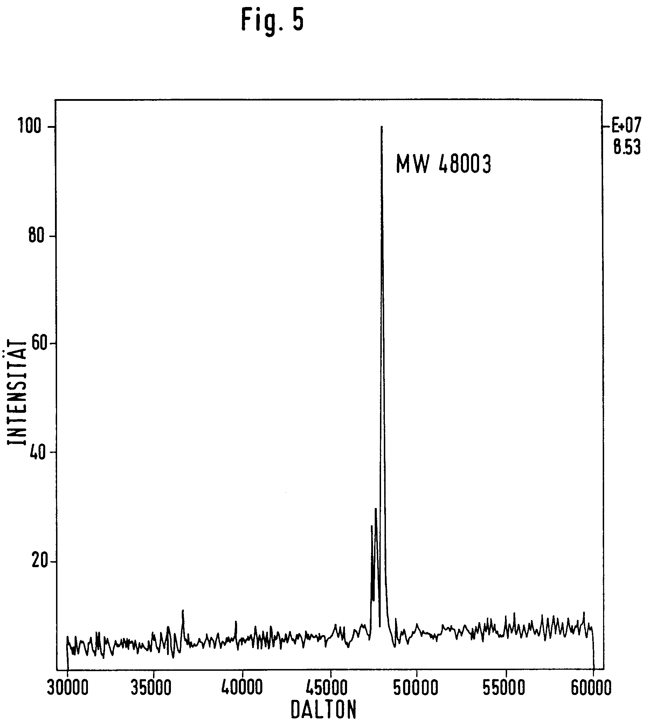

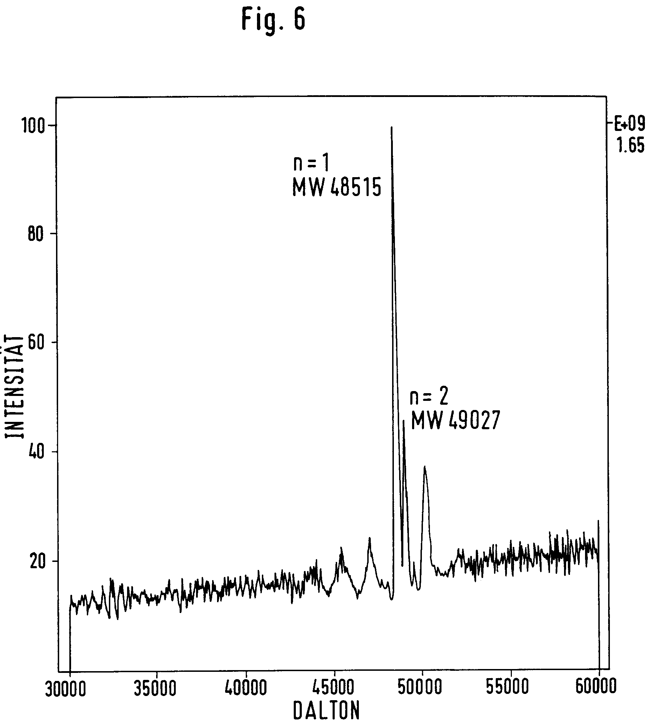

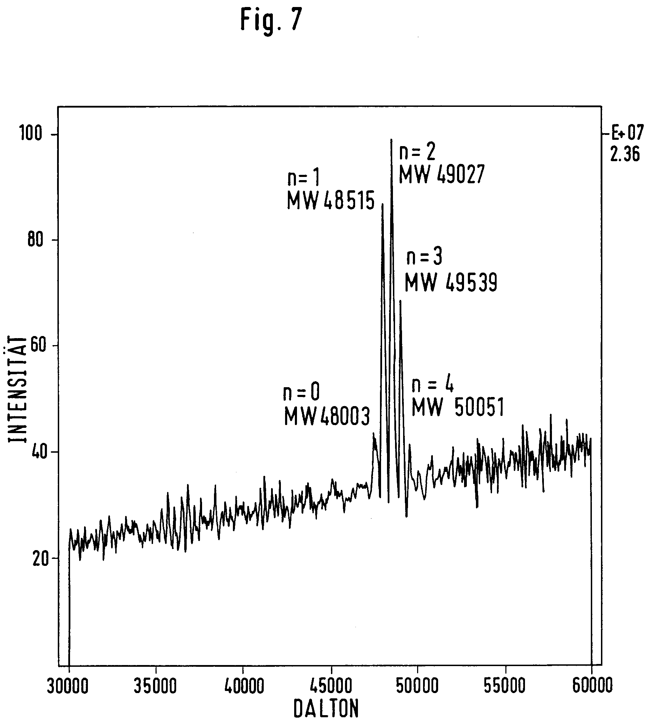

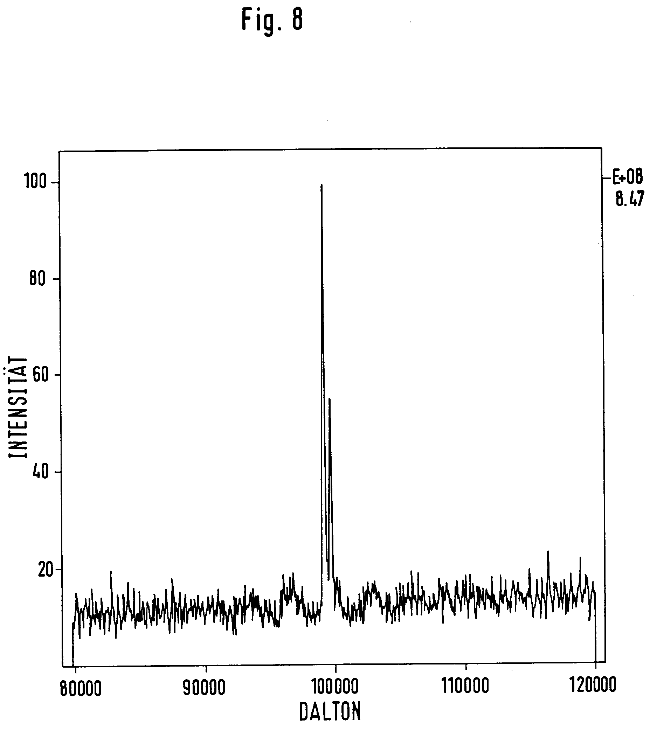

- MAK〈CEA〉 Fab nicht modifiziert: (Abb. 5)
- MAK〈CEA〉 Fab-(biotin)ₙ; n = 1, 2: (Abb. 6)
- MAK〈CEA〉 Fab-(biotin)ₙ; n = 0, 1, 2, 3, 4: (Abb. 7)
- MAK〈TSH〉 F(ab')₂-(biotin)ₙ; n = 1, 2: (Abb. 8)

## Patentansprüche

1. Verfahren zur quantitativen Bestimmung von Proteinen bzw. entsprechenden Derivaten in einer wäßrigen Probe, dadurch gekennzeichnet, daß die Messung massenspektrometrisch erfolgt und mindestens zwei Proteinkomponenten in einer Messung gleichzeitig bestimmt werden.

2. Verfahren nach Anspruch 1, dadurch gekennzeichnet, daß eine Probenvorbereitung, die in der Verdünnung der Probe mit einem für die Massenspektrometrie geeignetem Lösungsmittel besteht, durchgeführt wird.

3. Verfahren nach Anspruch 1 oder 2, dadurch gekennzeichnet, daß die Probe vor der Messung mit einem proteolytischem Enzym behandelt wird.

4. Verfahren nach einem der Ansprüche 1 bis 3, dadurch gekennzeichnet, daß die Probe vor der massenspektrometrischen Messung durch einem chromatographischen oder immunologischen Aufreinigungsverfahren unterworfen wird und die Aufreinigung gegebenenfalls on-line zur Massenspektrometrie erfolgt.

5. Verfahren nach einem der Ansprüche 1 bis 4, dadurch gekennzeichnet, daß zur massenspektronmetrischen Messung Verfahren wie "atmospheric pressure ionization", "electrospray ionization" oder ein anderes Spray-Verfahren oder ein Laserdesorptionsverfahren bzw. ein gleichwertiges Ionisationsverfahren verwendet werden.

6. Verfahren nach einem der Ansprüche 1 bis 5, dadurch gekennzeichnet, daß die Probe Albumin und dessen Derivate und/oder Hämoglobin und dessen Derivate und/oder unterschiedliche Antikörperfragmente enthält.

7. Verfahren nach einem der Ansprüche 1 bis 6, dadurch gekennzeichnet, daß die für die einzelnen Proteinkomponenten gewonnenen Meßdaten zueinander ins Verhältnis gesetzt werden und aus diesen Verhältniswerten diagnostische Aussagen ableitbar sind.

8. Verwendung des Verfahrens nach einem der Ansprüche 1 bis 7 für diagnostisch relevante Aussagen.
